# EUROPEAN PATENT SPECIFICATION

(11) **EP 0 448 940 B1**
(45) Date of publication and mention of the grant of the patent: **17.09.1997**
(21) Application number: 91101662.4
(22) Date of filing: 07.02.1991
(51) Int. Cl.: C07K 9/00, C12P 19/44, A61K 38/12, A61K 38/14, A23K 1/17

(54) **Process for the preparation of mannosyl teicoplanin derivatives and mannosyl teicoplanin aglycone**
Verfahren zur Herstellung von Mannosylteicoplaninderivaten und Mannosylteicoplaninaglykon
Procédé de préparation de dérivés de mannosyl-teicoplanin et de l'aglucone de mannosyl-teicoplanin

(30) Priority: 28.03.1990 EP 90105891
(43) Date of publication of application: 02.10.1991
(73) Proprietor: GRUPPO LEPETIT S.p.A., I-20020 Lainate (MI) (IT)
(72) Inventor: Borghi, Angelo, I-20124 Milano (IT); Lancini, Giancarlo, I-27100 Pavia (IT)
(74) Representative: Macchetta, Francesco

(56) References cited:
- EP-A- 0 146 053
- CHEMICAL ABSTRACTS, vol. 111, no. 19, 6 November 1989, Columbus, Ohio, US, abstract no. 172453B

## Description

The object of the present invention is a process for preparing a teicoplanin derivative of formula I 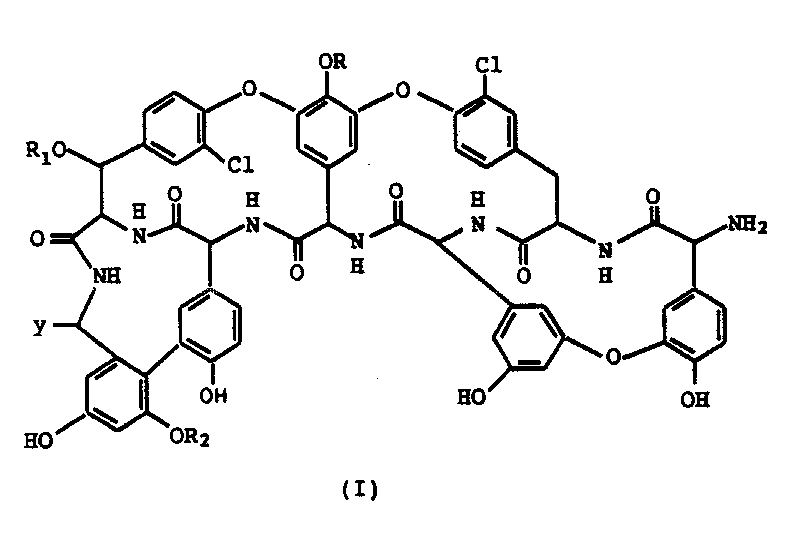 wherein
R is N-(Z-4-decenoyl)-β-D-2-deoxy-2-amino-glucopyranosyl, N-(8-methyl-nonanoyl)-β-D-2-deoxy-2-amino-glucopyranosyl, N-decanoyl-β-D-2-deoxy-2-amino-glucopyranosyl, N-(8-methyl-decanoyl)-β-D-2-deoxy-2-amino-glucopyranosyl, N-(9-methyl-decanoyl)-β-D-2-deoxy-2-amino-glucopyranosyl; or hydrogen;
R₁ is N-acetyl-β-D-2-deoxy-2-amino-glucopyranosyl;
R₂ is α-D-mannopyranosyl; with the proviso that R₁ represents hydrogen only when R is hydrogen;
Y is COOH; and the pharmaceutically acceptable addition salts thereof;
which comprises submitting a teicoplanin derivative of formula I wherein R and R₁ are defined as above and R₂ is hydrogen to a microbiological transformation with a culture of Actinoplanes teichomyceticus ATCC 31121, its natural mutants or variants thereof exhibiting the same property of forming the glycosidic bond between the D-mannose moiety and the hydroxylic function at position -OR₂ or hydrogen; of the teicoplanin starting material, the washed mycelium or a cell free preparation thereof.

A further object of this invention is the compound of formula I above wherein R and R₁ are both hydrogen atoms, R₂ is α-D-mannopyranosyl and Y is COOH. The mannosyl teicoplanin derivatives of this invention are antibiotically active compounds.

Teicoplanin is an antibiotic produced by cultivating the strain Actinoplanes teichomyceticus nov. sp. ATCC 31121 in a culture medium containing assimilable sources of carbon, nitrogen and inorganic salts.

The main product resulting from the above mentioned strain was a mixture of three main factors (A₁, A₂ and A₃) originally referred to as teichomycin (U.S. Patent 4,239,751).

The more recent teicoplanin preparations obtained by purification of the product recovered from the fermentation broth and suitable for chemotherapeutic use in the treatment of infections caused by gram-positive organisms (A.H. Williams et al.: Journal of Hospital Infection (1986); 7, Suppl. A, 101-103. D. Greenwood: Journal of Antimicrobial Chemotherapy (1988); 21, Suppl. A, 1-13) contain as the major component a complex of five structurally closely related substances which had been originally referred to, as whole, as teichomycin factor A₂. The above mentioned five closely related substances have been successively isolated and characterized as single components of the complex which was then currently designated and referred to in the scientific papers and patent literature as "teicoplanin A₂" or "teicoplanin complex".

The five major components of teicoplanin complex (conventionally named: TA2-1, TA2-2, TA2-3, TA2-4 and TA2-5) may be represented by the above general formula (I) wherein:

R respectively is:
- TA2-1):: N-(Z-4-decenoyl)-β-D-2-deoxy-2-aminoglucopyranosyl;
- TA2-2):: N-(8-methyl-nonanoyl)-β-D-2-deoxy-2-aminoglucopyranosyl;
- TA2-3):: N-decanoyl-β-D-2-deoxy-2-amino-glucopyranosyl;
- TA2-4):: N-(8-methyl-decanoyl)-β-D-2-deoxy-2-aminoglucopyranosyl;
- TA2-5):: N-(9-methyl-decanoyl)-β-D-2-deoxy-2-aminoglucopyranosyl;

- R₁ is: N-acetyl-β-D-2-deoxy-2-amino-glucopyranosyl;
- R₂ is: Alpha-D-mannopyranosyl;
- Y is: COOH

Their respective ratios in the teicoplanin complex can vary according to the fermentation conditions and the precursors added to the fermentation medium as described in the European Patent No.204179.

In the prior art are described the aglycone of teicoplanin (deglucoteicoplanin, L 17392), which is conventionally designated with the acronym TD and is one of the starting materials of the process of this invention, i.e. the compound of formula (I) above wherein R=R₁=R₂=hydrogen, Y=COOH, and two pseudo aglycones, namely compound L 17054 (formula I, R=hydrogen, R₁=N-acetyl-β-D-2-deoxy-2-aminoglucopyranosyl, R₂=alpha-D-mannopyranosyl Y=COOH, conventionally designated with the acronym TB) and compound L 17046 (formula I, R=R₂=hydrogen; R₁= N-acetyl-β-D-2-deoxy-2-amino-glucopyranosyl, Y=COOH, conventionally designated with the acronym TC), which is one of the starting materials of the process of the invention as well.

The above mentioned derivatives of teicoplanin are obtained by submitting the teicoplanin complex or the individual major components thereof to appropriate acid hydrolysis conditions. See for instance: European Patent Application publication No. 146053, European Patent No. 119575 and European Patent No. 119574.

In summary, mild acid hydrolysis conditions displace the acylglucosamine moiety (R), a stronger acidic treatment displaces the mannose unit (R₂) and a further acidic treatment allows displacement of the remaining N-acetyl-glucosamine moiety yielding the aglycone (R₁).

In conclusion the known hydrolysis conditions, displace first the sugar moiety R and then displace the sugar moiety R₂ and R₁ (in this order) and therefore do not permit to obtain the teicoplanin mannosyl aglycone (i.e. the compound of the formula I wherein R₂ is α-D-mannopyranosyl, R and R₁ being hydrogen atoms).

Therefore, according to the teaching of the prior-art it is particularly troublesome to find a process capable of providing teicoplanin pseudo aglycones wherein the mannosyl (R₂) rest is not accompanied by the simultaneous presence of the acetyl glucosamine (R₁) rest which is more strongly linked to the teicoplanin nucleus.

Other starting materials which can be used according to the process of the present invention are de-mannosyl teicoplanin derivatives, i.e. teicoplanin derivatives where R and R₁ have the same meanings as in the teicoplanin complex represented above and R₂ is hydrogen. These de-mannosylated teicoplanin derivatives can be obtained in good yields by microbiological transformation of a substrate selected from teicoplanin complex, any mixture of the single components and a single component thereof with cultures of Nocardia orientalis NRRL 2450 or Streptomyces candidus NRRL 3218, as described in European Patent Application publication No. 301247.

Samples of said strains bearing our internal codes A/156 and S/802 respectively have been redeposited on June 10, 1987 at the ATCC (American Type Culture Collection, 12301 Parklawn Drive, Rockville, MD 20852 U.S.A.) under the conditions established by the Budapest Treaty on the International Recognition of the Deposit of Microorganisms for the Purposes of Patent Procedure where they have been assigned the following ATCC numbers respectively 53630 and 53629.

In Journal of Antibiotic 39, May 1986 pag. 652, it is stated that the strain Actinoplanes teichomyceticus ATCC 31121 can be used to mannosylate the aglycone and the pseudoaglycone of the glycopeptidic antibiotic named aridicin, but nothing is said about the possibility to mannosylate a teicoplanin substrate.

Moreover in that publication it is disclosed that Actinoplanes teichomyceticus has different behaviours with respect to aridicin and teicoplanin. For instance, said strain can easily deacylate the aridicin substrate even if at the same time, it does not appear to deacylate any teicoplanin compound as well.

The strain Actinoplanes teichomyceticus ATCC 31121 is the teicoplanin producing strain and therefore this could suggest to a man skilled in the art that by contacting the teicoplanin aglycone or pseudo-aglycones with the above strain a complete glycosylation of the teicoplanin nucleus occurs instead of a specific mannosylation.

The mannosyl teicoplanin derivatives of this invention are prepared by submitting a substrate selected from teicoplanin complex, any mixture of the single components and a single component thereof which can be represented by the general formula I above wherein:
R respectively is:
N-(Z-4-decenoyl)-β-D-deoxy-2-aminoglucopyranosyl;
N-(8-methyl-nonanoyl)-β-D-2-deoxy-2-aminoglucopyranosyl;
N-decanoyl-β-D-2-deoxy-2-amino-glucopyranosyl; N-(8-methyl-decanoyl-β-D-2-deoxy-2-aminoglucopyranosyl;
N-(9-methyl-decanoyl)-β-D-2-deoxy-2-aminoglucopyranosyl; or hydrogen;

R₁ is N-acetyl-β-D-2-deoxy-2-amino-glucopyranosyl; or hydrogen;
R₂ is hydrogen, with the proviso that R₁ represents hydrogen only when R is hydrogen; Y is COOH; to a micro-biological transformation with a microorganism selected from strain Actinoplanes teichomyceticus sp. ATCC 31121, variants and mutants thereof exhibiting the same property of forming the glycosidic bond between the D-mannose moiety and the hydroxylic function of teicoplanin starting materials, the washed mycelium and a cell-free preparation thereof.

The expression "mutants and variants thereof" as used here in the description and claims refers to those mutants and variants of Actinoplanes teichomyceticus ATCC 31121 which show substantially the same morphological and physiological characteristics as the parent strain Actinoplanes teichomyceticus ATCC 31121 and which have the same property of linking the D-mannose moiety to the hydroxylic function of the teicoplanin molecule. According to a preferred embodiment of this invention, the selected starting material either in pure form or in the form of any crude preparation thereof is contacted with a growing culture of the above strain under fermentation conditions.

For the sake of brevity each de-mannosyl teicoplanin compound starting material of this invention will be hereinafter indicated with a conventional name referring to the teicoplanin complex major component from
which it derives, preceded by the acronym DM.
Accordingly:
DM-TA2-1 indicates the de-mannosyl derivative of component 1 (TA2-1);
DM-TA2-2 indicates the de-mannosyl derivative of component 2 (TA2-2);
DM-TA2-3 indicates the de-mannosyl derivative of component 3 (TA2-3);
DM-TA2-4 indicates the de-mannosyl derivative of component 4 (TA2-4);
DM-TA2-5 indicates the de-mannosyl derivative of component 5 (TA2-5).

The process of the present invention comprises also all those cases in which the starting materials are formed by a mixture of the compounds having the meanings of R described above i.e. a mixture of the DM-TA2 components defined above.
Since the mannosylating strain of the invention (i.e. Actinoplanes teichomyceticus ATCC 31121) is also the teicoplanin producing strain it is clear that if the starting material is one of the single DM-TA2 components the final result of the process is an enrichment of the corresponding TA2 component.

If the starting material is a mixture of the five DM-TA2 components the final result is an enrichment of all the five TA2 major components of the teicoplanin complex.

In the same way if the compound L 17046 (i.e. the pseudo-aglycone defined by the acronym TC cited above) is used as starting material of the process of the invention the final product mixture is the teicoplanin accompanied by the complex more polar teicoplanin-like product designated as TA-3-1 (see Malabarba et al., Journal of Antibiotics, Vol. XXXVII, No. 9, pag. 989-999) which is the compound L 17054 defined above by the acronym TB.

The method of obtainment of such mixtures of compounds identified by the formula (I) above is also an object of this invention. If desired the separation of each single component of the resulting mixtures may be easily carried out as it is well known in the art, for instance by the procedures described in USP 4,542,018.

The above mentioned strain is cultivated under usual submerged aerobic conditions in a medium containing assimilable sources of carbon, nitrogen and inorganic salts as described in US Patent no. 4,239,751.

Generally, the starting material mentioned above can be added to a culture of Actinoplanes teichomyceticus sp. ATCC 31121 at a time varying from 18 hours from the inoculation time to the time at which the culture has reached its maximum growth, however, addition after 24-72 hours from inoculation is, at least in some instances, preferred.

The reaction time, i.e. the time of exposure of the starting material to the microbial culture before recovering the final product, may vary between 4 and 48 hours, depending on the specific conditions employed. Anyway, since the reaction can be monitored as known in the art, for instance by following the decrease of the starting material and/or the increase of the final product by HPLC, the skilled man is capable of readily determine when the reaction is to be considered as complete and the recovery procedure can be started.

The temperature of the microbiological mannosylation is usually ranging from 25°C to 35°C and preferably about 28°C. Instead of employing a growing culture of Actinoplanes teichomyceticus sp. ATCC 31121 one may employ a culture of any mutant or variant thereof which is still capable of forming the glycosidic bond between the phenolic moiety and the mannose portion of the above mentioned starting material to give the mannosylated compounds of the invention. Any process according to the present invention which employs any such mutant or variant, is considered to be encompassed by the scope of the present invention.

Moreover, the compounds of the present invention can be prepared according to the method of the invention by using a mycelium of the above identified mannosylating microorganism culture, washed in an isotonic saline solution, conveniently NaCl, in order not to disrupt said mycelium.

The mycelium can be preferably collected by centrifugation and the washing procedure is preferably repeated three times.

After having washed the mycelium, it is conveniently resuspended in a physiologically acceptable medium. The washed mycelium procedure can be used in order to increase the amounts of teicoplanin compounds to be produced while maintaining optimal yields.

In fact it is known that the antibiotic production occurs after the complete growth of the microorganism so that it starts to produce the antibiotic only after the mycelium has been washed and resuspended. By means of this known technique it is easier to separate the final product from the nutrients and other biological material present in the fermentation broth thus simplifying the recovery and avoiding loss of product.

It is also possible to use a cell-free preparation by disrupting the cells, e.g. by sonication.

The recovery of the antibiotic substances from the reaction medium is then conducted according to known per sè techniques which include extraction with solvents, precipitation by adding non-solvents or by changing the pH of the solution, partition chromatography, reverse-phase partition chromatography, ion-exchange chromatography, affinity chromatography and the like.

A preferred procedure includes an affinity chromatography on immobilized D-Alanyl-D-Alanine followed by separation at different pH values.

Immobilized D-Alanyl-D-Alanine matrices suitable for the present recovery process are disclosed in European Patent No. 122969. The preferred matrix in this recovery process is D-Alanyl-D-Alanine coupled with a controlled pore cross-linked polydextrane.

The reaction medium can be subjected to the affinity chromatography directly after filtration or after a preliminary purification procedure. This latter procedure includes making the whole medium basic, preferably between pH 8.5 and 11 and then filtering in the presence of a filter aid, if convenient.

The clear filtrate is then adjusted to a pH value between 7 and 8 and then subjected to an affinity chromatography on immobilized D-alanyl-D-alanine, either in column or batchwise.

While the binding of the substance to the affinity matrix is preferably made at a pH of about 7.0-8.0, its elution is performed at more basic pH values (preferably between 9.0 and 10.5) by means of an aqueous base. This aqueous base may be ammonia, a volatile amine, an alkali or alkali metal hydroxide or a basic buffered solution optionally in the presence of a polar organic solvent such as a polar water-miscible solvent.

Representative examples of polar water-miscible solvents are: lower aliphatic alcohols, (such as methanol, ethanol, iso-propanol, n-butanol), acetone, acetonitrile, lower alkyl alkanoates (such as ethyl acetate), tetrahydrofuran, dioxane and dimethylformamide and mixtures thereof; the preferred polar water-miscible solvent being acetonitrile.

After removing the impurities by rinsing the column with aqueous buffer pH 4-9, optionally containing salts, (e.g. ammonium formate) urea and/or water-miscible solvents, the mannosyl teicoplanin antibiotic substance is eluted with the above eluting mixture. The eluate is analyzed by HPLC and the fractions containing the desired material are pooled together.

This eluate is adjusted to pH 7.0-7.5 with an organic or mineral acid.

The eluate is then submitted to concentration and desalting procedures.

A convenient convenient desalting procedure includes applying the antibiotic containing aqueous solution to a silanised silica gel column, washing with distilled water and eluting with a mixture of a polar water-miscible solvent as defined above and water.

Alternatively, the aqueous solution of the mannosylated teicoplanin derivative(s) is submitted to simultaneous concentration desalting procedures by ultrafiltration through a ultrafiltration membrane with a nominal molecular weight limit (NMWL) of 1000 dalton or less.

The solution obtained from the above procedure is then lyophilized and the recovered material is submitted to further purification.

In some cases, in particular, for large scale preparations, it is preferred to carry out said purification in two steps. The first one is carried out according to a reverse phase chromatography general procedure already described in US Patent No; 4,542,018 for the separation of the individual factors of teicoplanin complex. According to a specific embodiment of said procedure, the mannosyl teicoplanin derivative(s) product obtained from lyophilization is dissolved in an acetonitrile/ammonium formate mixture and adjusted to pH 7.5 with sodium hydroxide and the obtained solution is passed through a silanised silica gel column and then the column is eluted with a linear gradient of acetonitrile in ammonium formate solution.

The eluate is monitored by HPLC and the fractions containing the desired material(s) are pooled together and evaporated under reduced pressure yielding the solid material desired. This procedure is also useful for the separation of the single mannosyl derivatives of teicoplanin when this latter or a mixture of its single components is used as the starting material instead of the individual components.

The first purification step may be avoided when the starting material utilized for the microbiological transformation is sufficiently pure and essentially consists of an individual component of de-mannosyl teicoplanin mixture.

The second purification step involves a semi-preparative HPLC on a silanised chemically modified preparative HPLC column by using two mixtures of acetonitrile/ammonium formate in different ratios as mobile phases and maintaining a linear gradient of acetonitrile in ammonium formate. The eluted fractions are monitored by HPLC analysis and those containing the desired product are pooled together, the organic solvent is evaporated under reduced pressure and then the aqueous solution is submitted to simultaneous concentration/desaltion by ultrafiltration as described above. The solution resulting from ultrafiltration is then lyophilized yielding the desired pure product.

The mannosyl teicoplanin derivatives of this invention are active against gram-positive bacteria which are responsible for many widely diffused infections.

As described above a further object of the present invention is the compound of formula I wherein R and R₁ are hydrogen atoms, R₂ is α-D-mannopyranosyl and Y is COOH (conventionally named MTD).

The anti-bacterial activity of the compound of the invention can be demonstrated in vitro by means of standard dilution tests on different microorganism cultures.

The antibacterial activity of the mannosyl teicoplanin aglycone (MTD) against some strains is surprisingly higher than the anti-bacterial activity of the other known pseudoaglycones of teicoplanin; i.e. the other derivatives of teicoplanin having at least one of the three characteristic sugar moieties.

Culture media and growth conditions for MIC (minimal inhibitory concentration) determinations were as follows: Isosensitest broth (Oxoid), 24 h, for staphylococci, Strep. faecalis and Gram-negative bacteria (Escherichia coli) ; Todd-Hewitt broth (Difco), 24 h for other streptococcal species; GC base broth (Difco) + 1% Isovitalex (BBL), 48 h, CO₂-enriched atmosphere for Neisseria gonorrhoeae; Brain Heart broth (Difco) + 1% Supplement C (Difco), 48 h for Haemophilus influenzae; Inocula were of about 10⁴-10⁵ colony-forming units/ml for broth dilution MICs.

The minimal inhibitory concentrations (MIC, microgram/ml) of the above de-mannosyl teicoplanin derivatives and of the other pseudo aglycones for some microorganisms are reported below in Table I.

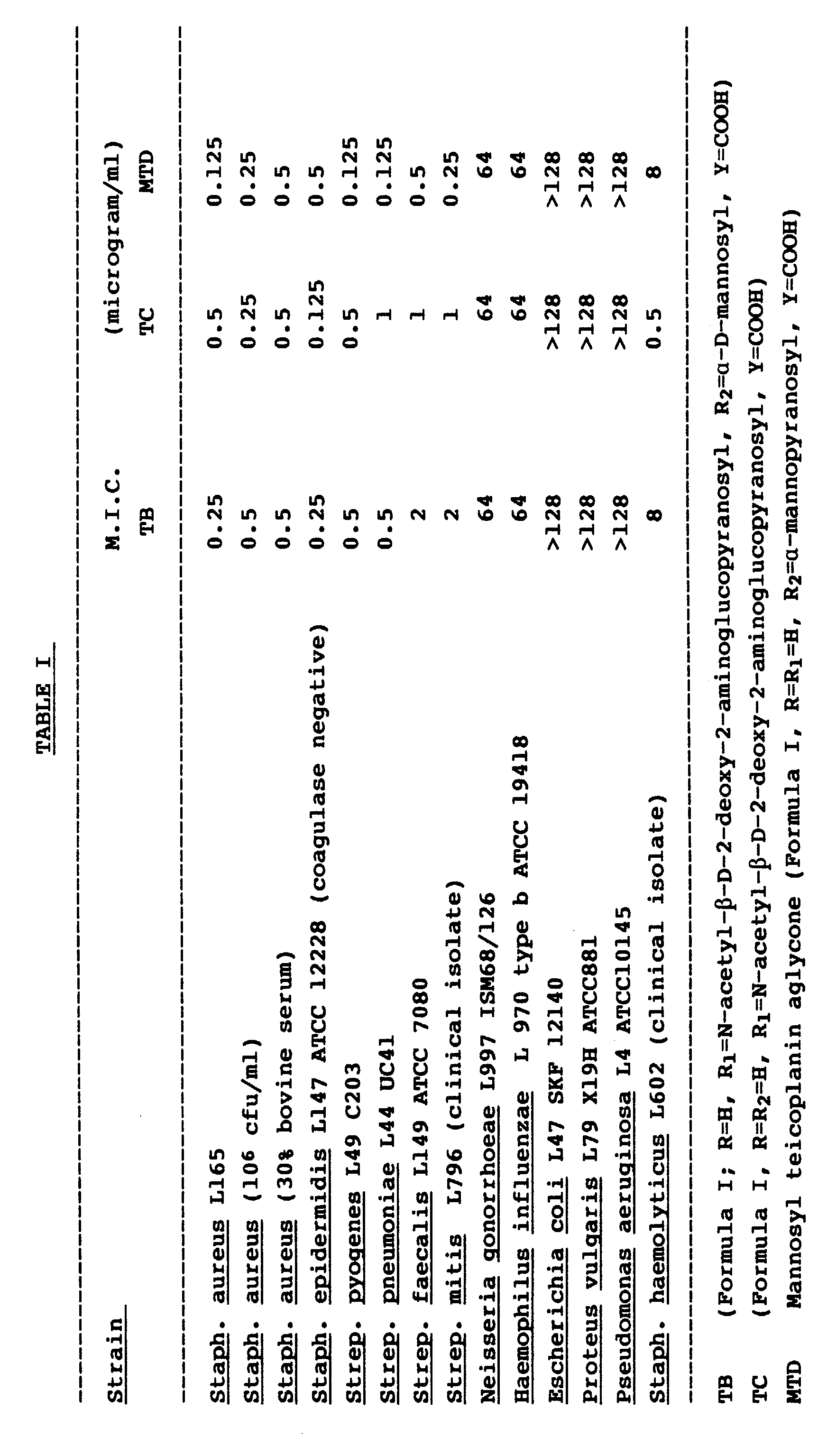

The mannosyl teicoplanin aglycone possesses acid and basic functions and can form salts with organic and inorganic counter ions according to conventional procedures.

Representative and suitable acid addition salts of the compound of the invention include those salts formed by standard reaction with both organic and inorganic acids such as, for example, hydrochloric, hydrobromic, sulfuric, phosphoric, acetic, trifluoroacetic, trichloroacetic, succinic, citric, ascorbic, lactic, maleic, fumaric, palmitic, cholic, pamoic, mucic, glutamic, camphoric, glutaric, glycolic, phthalic, tartaric, lauric, stearic, salicylic, methanesulfonic, benzenesulfonic, sorbic, picric, benzoic, cinnamic and the like acids.

Representative examples of bases are: alkali metal or alkaline-earth metal hydroxydes such as sodium, potassium, calcium, magnesium, barium hydroxide; ammonia and aliphatic, alicyclic or aromatic organic amines such as methylamine, dimethylamine, trimethylamine, and picoline.

The transformation of the "non-salt" compounds of the invention into the corresponding addition salts, and the reverse, i.e. the transformation of an addition salt of a compound of the invention into the non-salt form, are within the ordinary technical skill and are encompassed by the present invention.

For instance, mannosyl teicoplanin aglycone can be transformed into the corresponding acid or base addition-salt by dissolving the non-salt form in an aqueous solvent and adding a slight molar excess of the selected acid or base. The resulting solution or suspension is then lyophilized to recover the desired salt.

In case the final salt is insoluble in a solvent where the non-salt form is soluble it is recovered by filtration from the organic solution of the non-salt form after addition of the stoichiometric amount or a slight molar excess of the selected acid or base.

Examples of these insoluble salts are calcium, magnesium and barium salts.

The non-salt form can be prepared from a corresponding acid or base salt dissolved in an aqueous solvent which is then neutralized to free the non-salt form.

When following the neutralization the elimination of the excess of acid or base is necessary, a common desalting procedure may be employed.

For example, column chromatography on silanised silica gel, non-functionalized polystyrene, acrylic and controlled pore polydextrane resins (such as Sephadex LH 20) or activated carbon may be conveniently used. After eluting the undesired salts with an aqueous solution, the desired product is eluted by means of a linear gradient or a step-gradient of a mixture of water and a polar or apolar organic solvent, such as acetonitrile/water from 50:50 to about 100% acetonitrile.

As it is known in the art, the salt formation either with pharmaceutically acceptable acids (or bases) or non-pharmaceutically acceptable acids (or bases) may be used as a convenient purification technique. After formation and isolation, the salt form of the mannosyl teicoplanin aglycone can be transformed into the corresponding non-salt form or into a pharmaceutically acceptable salt form. In general, for the antibacterial treatment the mannosyl teicoplanin aglycone as well as the non-toxic pharmaceutically acceptable salts thereof or mixture thereof, can be administered by different routes such as topically or parenterally. The parenteral administration is, in general, the preferred route of administration.

Compositions for injection may take such forms as suspensions, solutions, or emulsions in oily or aqueous vehicles, and may contain adjuvants such as suspending, stabilizing and/or dispersing agents.

Alternatively, the active ingredient may be in powder form for reconstitution at the time of delivery when a suitable vehicle, such as sterile water, is added thereto.

Depending on the route of administration, this compound can be formulated into various dosage forms.

In some instances, it may be possible to formulate the compounds of the invention in enteric-coated dosage forms for oral administration which may be prepared as known in the art (see for instance "Remington's Pharmaceutical Sciences", fifteenth edition, Mack Publishing Company, Easton, Pennsylvania, USA, page 1614).

This could be specially the case when the absorption of the antimicrobial substance in the enteric tract is particularly desired while passing unaltered through the gastric tract.

The amount of active principle to be administered depends on various factors such as the size and condition of the subject to be treated, the route and frequency of administration, and the causative agent involved.

The antibiotic substance of the present invention and the physiologically acceptable salts thereof, is generally effective at a daily dosage of between about 0.5 and 50 mg of active ingredient per kilogram of patient body weight, optionally divided into 1 to 4 administrations per day.

Particularly desirable compositions are those prepared in dosage units containing from about 50 to about 2,000 mg per unit.

Sustained-action formulations can be prepared based on different mechanisms and methods, as known in the art.

A preferred method for preparing a sustained-action formulation containing the mannosyl antibiotic substance, involves the use of a water insoluble form of the antibiotic suspended in an aqueous or oily medium.

Besides their activity as medicaments, the mannosyl antibiotic of this invention and the non-toxic salts thereof, can be used as animal growth promoters, i.e. to increase the feed efficiency of meat or milk producing animals.

For this purpose, the compound of the invention is administered orally in a suitable feed. The exact concentration employed is that which is required to provide for the active agent in a growth promotant effective amount when normal amounts of feed are consumed.

The addition of the active compound of the invention to animal feed is preferably accomplished by preparing an appropriate feed premix containing the active compound in an effective amount and incorporating the premix into the complete ration.

Alternatively, an intermediate concentrate or feed supplement containing the active ingredient can be blended into the feed.

The way in which such feed premixes and complete rations can be prepared and administered are described in reference books (such as "Applied Animal Nutrition", W.H. Freedman and CO., S. Francisco, USA, 1969 or "Livestock Feeds and Feeding" O and B books, Corvallis, Oregon, USA, 1977) and are incorporated herein by reference.

The following examples further illustrate the invention and must not be construed as limiting it.

### Detailed description of the preparation of the mannosyl teicoplanin derivatives

### 1. Preparation of teicoplanin mannosyl-aglycone.

A vial containing lyophilized Actinoplanes teichomyceticus ATCC 31121 was opened and the microorganism was aseptically transferred into a slant of oatmeal agar. After a 7 day incubation at 28°C, the culture was suspended in distilled water and inoculated into 2 Erlenmeyer flasks each containing 100 ml of vegetative medium S/bis having the following composition:

| | |
|---|---|
| Yeast extract | 4 g |
| Peptone | 4 g |
| Glucose | 10 g |
| MgSO₄ | 0.5 g |
| KH₂PO₄ | 2 g |
| K₂HPO₄ | 4 g |
| Distilled water to | 1000 ml |
| pH after sterilization: | 7 |

The inoculated medium was incubated 48 hours at 28°C on a rotary shaker at 200 rpm. The resulting culture, subdivided in several portions of 5 ml each, was frozen and stored for further use.

A portion of 2.5 ml of the frozen stock culture was used to inoculate a 500 ml Erlenmeyer flask containing 100 ml of vegetative medium S/bis. The culture was incubated at 28°C for 48 h on a shaker at 200 rpm and 5 cm throw.

Five ml of this culture were used to inoculate 100 ml of productive medium C in a 500 ml flask having the following compositions:

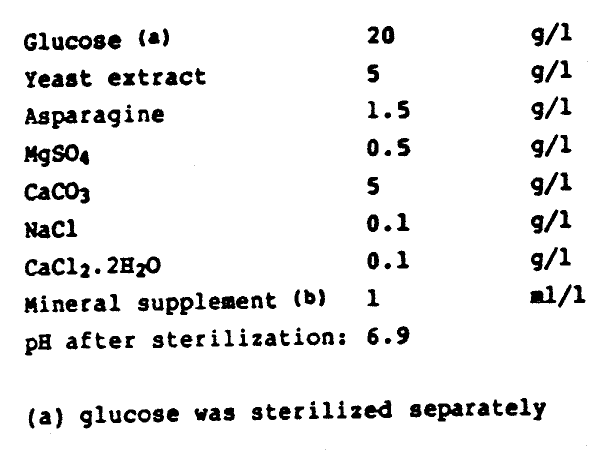

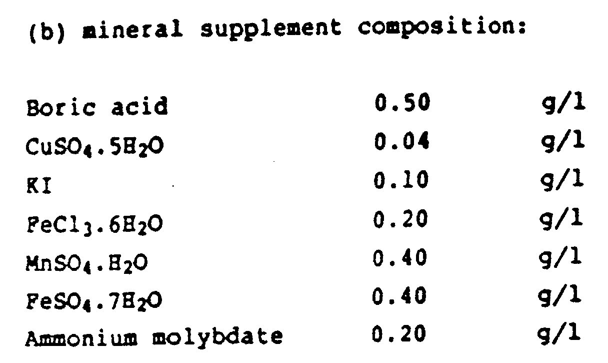

Ten flasks were prepared according to the procedure described above. Fourty hours after inoculation, the mycelium was collected by centrifugation and washed three times with 500 ml of sterile saline. The cells were then suspended in 1 liter of sterile saline containing 200 mg of teicoplanin aglycone and distributed in 10 flasks (100 ml each). The transformation was carried out at 28°C on a rotary shaker at 200 rpm for 24 hours.

The whole reaction medium from all ten flasks was brought to pH 10.5 by addition of 1 N NaOH and then filtered in the presence of a filter aid. The pH of the filtered broth was adjusted to 7.5 by adding 1 N HCl and 50 ml of Sepharose-epsilon-aminocaproyl-D-alanyl-D-alanine affinity resin (European Patent No.122969) are added thereto.

The mixture was stirred overnight at 4°C. The resin was then separated from the exhausted broth and poured into a chromatographic column. The column was washed with five resin volumes of Tris-HCl buffer (0.05 M, pH 7.5) and then with the one resin volume of Tris base solution (0.05 M). The resin was eluted with a solution of 1% ammonium hydroxide collecting several fractions of 10 ml each. Fractions were neutralized with formic acid and analyzed by HPLC. The HPLC analysis was carried out under the following conditions:
Instrument: Hewlett Packard model 1084 B with a 254 nm detector;
Column: Erbasil C-18, 5 micrometer, 4.6 x 150 mm;
Mobile phases:
A) CH₃CN:NaH₂PO₄ (0.02 M), 5:95;
B) CH₃CN:NaH₂PO₄ (0.02 M), 75:25;

| Gradient profile as follows: | | | | | |
|---|---|---|---|---|---|
| min | 0 | 40 | 45 | 48 | 50 |
| % B | 8 | 40 | 55 | 8 | stop |

Flow rate: 1.5 ml/min;
Injection: e.g. 20 microliter of a solution of test substance at about 1 mg/ml in H₂O or H₂O:CH₃CN, 1:1.

Under the above conditions teicoplanin aglycone showed a retention time (Rt) of 12.80 min while teicoplanin mannosyl-aglycone showed a Rt of 11.1 minutes.

The fractions from affinity column containing mannosyl-aglycone were combined (about 60 ml) and then concentrated by ultrafiltration by using a 60 mm AMICON ^{R} ultrafiltration cell supporting a ultrafiltration membrane with a nominal molecular weight limit (NMWL) of 1000 dalton. The volume of the solution was reduced to about 5 ml and the residual is lyophilized giving 252 mg of crude product.

The crude product was further purified by semi-preparative HPLC under the folloving conditions:
Apparatus: Waters liquid chromatograph, equipped with two pumps model 6000A, an absorbance UV detector model 440 set at 254 nm and a solvent programmer model 660.
Column: HIBAR LiChrosorb RP-18, 7 micrometer, 250 x 10 mm (Merck);

Mobile phase:
A) aqueous (2 g/l) HCOONH₄ / CH₃CN (9:1)
B) aqueous (2 g/l) HCOONH₄ / CH₃CN (3:7);

Gradient: linear from 5% of B to 45% of B in 45 m minutes
Flow rate: 6 ml/min
Injection: 10 mg of product dissolved in 2 ml of A each time.

The portions of eluate which contain teicoplanin mannosyl-aglycone identified through the chromatographic profile, were collected.

The above described semi-preparative purification was applied to the whole crude product recovered from ultrafiltration and the eluate portions were combined (165 ml as a whole) and the organic solvent was evaporated under vacuum. The remaining aqueous solution of mannosyl aglycone was concentrated and desalted by ultrafiltration under the same conditions to about 5 ml and the remaining solution was lyophilized yielding 114 mg of pure mannosyl-aglycone, i.e. the compound of formula I above wherein: R is hydrogen, R₁ is hydrogen R₂ is α-D-mannopyranosyl, and Y is COOH.

The 1H-NMR spectrum of the pure teicoplanin mannosyl-aglycone was recorded using a Bruker ^{R} model AM-250 instrument with an array processor, a magnet at 250 MHz, and a computerized console Aspect 3000. The spectra were obtained for protons in DMSO-d₆ solutions at 25°C with TMS as reference.

The most significative signals of MTD in comparison with those of the antibiotic L 17046 (TC) are reported in Table II. UV data of mannosyl teicoplanin aglycone are reported in Table III.

The Fast Atom Bombardment Mass Spectrum (FAB) of the pure teicoplanin mannosyl-aglycone was recorded with a VG apparatus model 70-70 EQ equipped with a FAB source. The positive ion spectra were obtained from the samples dispersed in a few microliters of alpha-thioglycerol, bombarded with a 7 KeV beam of Ar atoms. This experiment indicates a molecular weight of 1359 which is consistent with the structure assigned.

**TABLE II**

| ¹H-NMR data (δ, ppm) of mannosylaglycon at 250 MH_{z} in DMSOd₆. | | |
|---|---|---|
| PROTON | MTD | TC |
| CH₂ of mannose | 3.50 | absent |
| X₆, X₅, X₇, X₁ | 4.51-4.15 | 4.60-4.13 |
| X₂ | 4.85 | 4.97 |
| anomeric proton of acetyl glucosamine | absent | 4.38 |
| acetyl group of acetyl glucosamine | absent | 1.87 |
| 4_{F}, Z₆, X₃ | 5.31-5.09 | 5.34-5.10 |
| anomeric proton of mannose | 5.31-5.09 | about 5 |
| 4b | 5.59 | 5.57 |
| X₄ | 5.83 | 5.60 |
| Z₆OH | 5.90 | absent |
| aromatic protons NH's and phenolic OH's | 6.20 | 6.29-10.0 |

Nomenclature and symbols used in Table II above are those proposed in the following reference: D.H. Williams et al., J. Am. Chem. Soc. 1984, 106, 4895-4902 (see in particular Figure 4 on page 4899).

**TABLE III**

| U.V. Data (λₘₐₓ (nm) | |
|---|---|
| SOLVENT | λₘₐₓ (nm) |
| pH 5.9 buffer | 278 |
| KOH O.1N | 296 |

## Claims

1. A process for preparing a teicoplanin derivative of formula I 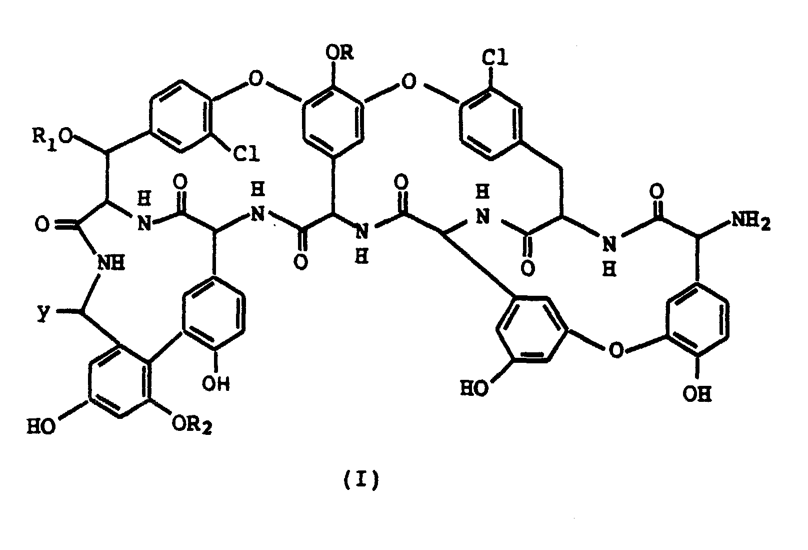 wherein
R is N-(Z-4-decenoyl)-β-D-2-deoxy-2-amino-glucopyranosyl, N-(8-methyl-nonanoyl)-β-D-2-deoxy-2-amino-glucopyranosyl, N-decanoyl-β-D-2-deoxy-2-amino-glucopyranosyl, N-(8-methyl-decanoyl)-β-D-2-deoxy-2-amino-glucopyranosyl, N-(9-methyl-decanoyl)-β-D-2-deoxy-2-amino-glucopyranosyl; or hydrogen
R₁ is N-acetyl-β-D-2-deoxy-2-amino-glucopyranosyl; or hydrogen
R₂ is α-D-mannopyranosyl; with the proviso that R₁ represents hydrogen only when R is hydrogen; Y is COOH and the pharmaceutically acceptable addition salts thereof;
which comprises submitting a teicoplanin derivative of formula I wherein R and R₁ are defined as above and R₂ is hydrogen to a microbiological transformation with a culture of Actinoplanes teichomyceticus ATCC 31121, its natural mutants or variants thereof exhibiting the same property of forming the glycosidic bond between the D-mannose moiety and the hydroxylic function at position -OR₂ of the teicoplanin starting material, the washed mycelium or a cell free preparation thereof and recovering said substance or mixture of substances and, in case a mixture of substances is obtained, optionally separating it into its individual components.

2. A process of claim 1 wherein the microbial transformation is carried out by contacting the substrate with a growing culture of the above strain cultivated under submerged aerobic conditions in a medium containing assimilable sources of carbon, nitrogen and inorganic salts at a temperature ranging from 25°C to 35°C for a period of time of 4 to 48 hours and the recovery of the substances and/or the optional separation of the mixture thereof, is carried out according to separation and purification procedures known per se in the art.

3. A process of claim 1 and 2 wherein is used mycelium of the above identified mannosylating microorganism culture, washed in an isotonic saline solution.

4. The compound of the formula I wherein R and R₁ are hydrogen atoms, R₂ is α-D-mannopyranosyl, and Y is COOH.

5. The compound of claim 4 for use as a medicament.

6. Use of the compound of claim 4 as animal growth factor.

7. A pharmaceutical composition comprising an antibacterially effective amount of the antibiotic compound of claim 4.

8. An animal feed composition comprising a non toxic amount of the antibiotic of claim 4 which is effective as growth promoter.

## Patentansprüche

1. Verfahren zur Herstellung eines Teicoplaninderivates der Formel I 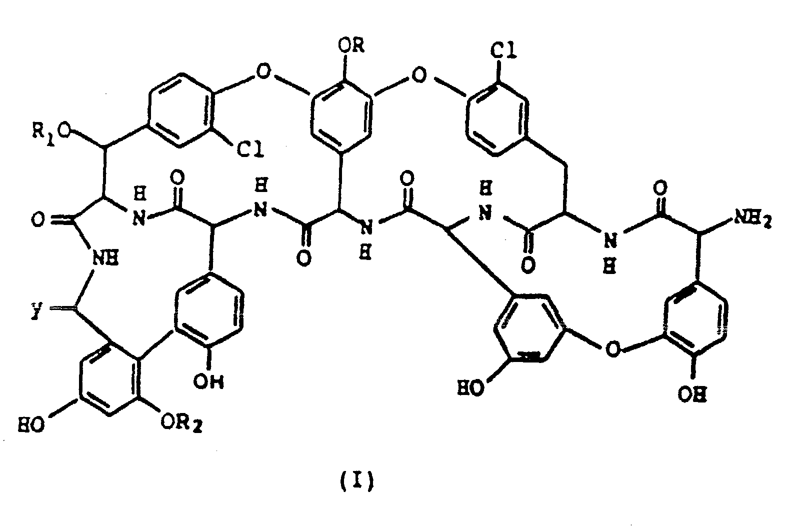 wobei:
R N-(Z-4-Decenoyl)-β-D-2-desoxy-2-aminoglucopyranosyl, N-(8-Methylnonanoyl)-β-D-2-desoxy-2-aminoglucopyranosyl, N-Decanoyl-β-D-2-desoxy-2-aminoglucopyranosyl, N-(8-Methyldecanoyl)-β-D-2-desoxy-2-aminoglucopyranosyl, N-(9-Methyl-decanoyl)-β-D-2-desoxy-2-aminoglucopyranosyl; oder Wasserstoff ist ;
R₁ N-Acetyl-β-D-2-desoxy-2-aminoglucopyranosyl; oder Wasserstoff ist;
R₂ ein α-D-Mannopyranosyl ist; mit der Maßgabe, daß R₁ nur dann Wasserstoff darstellt, wenn R Wasserstoff ist;
Y COOH ist; und deren pharmazeutisch verträgliche Additionssalze;
umfassend:
Unterziehen eines Teicoplaninderivates der Formel I, wobei R und R₁ wie vorstehend definiert sind und R2 Wasserstoff ist, einer mikrobiologischen Transformation mit einer Kultur von Actinoplanes teichomyceticus ATCC 31121, seinen natürlichen Mutanten oder deren Varianten, die die gleiche Eigenschaft der Erzeugung der glykosidischen Bindung zwischen der D-Mannoseeinheit und der Hydroxylfunktion an der Stellung -OR₂ des Teicoplanin-Ausgangsmaterial zeigen, dem gewaschenen Myzel oder einer zellfreien Präparation davon und Gewinnung dieser Substanz oder des Gemisches von Substanzen und im Fall, daß ein Gemisch von Substanzen erhalten wird, gegebenenfalls Trennen in seine einzelnen Komponenten.

2. Verfahren nach Anspruch 1, wobei die Mikroorganismus-Transformation durchgeführt wird durch Kontaktieren des Substrates mit einer wachsenden Kultur des vorstehenden Stammes, der unter submersen aeroben Bedingungen in einem Medium gezüchtet wird, das assimilierbare Quellen von Kohlenstoff, Stickstoff und anorganischen Salzen enthält, bei einer Temperatur von 25°C bis 35°C während eines Zeitraums von 4 bis 48 Stunden und die Gewinnung der Substanzen und/oder die fakultative Trennung des Gemischs daraus nach im Fachgebiet per se bekannten Trennungs- und Reinigungsverfahren durchgeführt wird.

3. Verfahren nach Anspruch 1 und 2, wobei ein Myzel der vorstehend identifizierten mannosylierenden Mikroorganismuskultur, gewaschen in einer isotonischen Salzlösung, verwendet wird.

4. Verbindung der Formel I, wobei R und R₁ Wasserstoffatome sind, R₂ α-D-Mannopyranosyl ist und Y COOH ist.

5. Verbindung nach Anspruch 4 zur Verwendung als Medikament.

6. Verwendung der Verbindung nach Anspruch 4 als Wachstumsfaktor für Tiere.

7. Arzneimittel, umfassend eine antibakteriell wirksame Menge der Antibiotikaverbindung nach Anspruch 4.

8. Tierfutterzusammensetzung, umfassend eine nichttoxische Menge des Antibiotikums nach Anspruch 4, die als Wachstumsförderer wirksam ist.

## Revendications

1. Procédé pour préparer un dérivé de teicoplanine de formule I 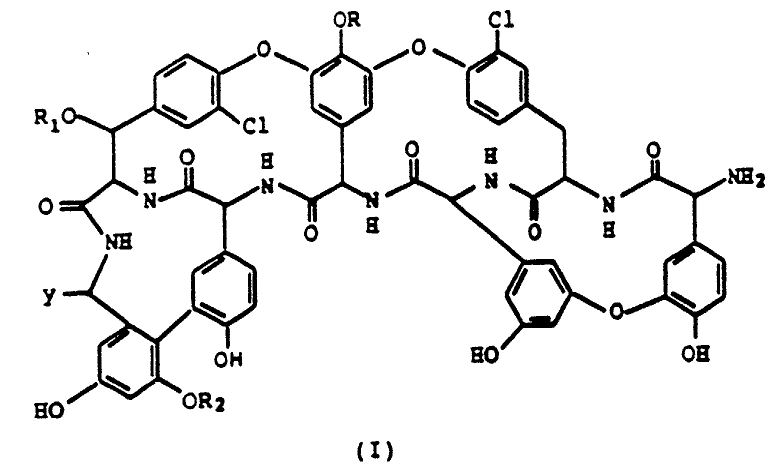 dans laquelle
R est un groupe N-(Z-4-décénoyl)-β-D-2-désoxy-2-aminoglucopyrannosyle, N-(8-méthyl-nonanoyl)-β-D-2-désoxy-2-amino-glucopyrannosyle, N-décanoyle-β-D-2-désoxy-2-amino-glucopyrannosyle, N-(8-méthyl-décanoyl)-β-D-2-désoxy-2-amino-glucopyrannosyle ou N-(9-méthyldécanoyl)-β-D-2-désoxy-2-amino-glucopyrannosyle; ou un atome d'hydrogène ;
R₁ est un groupe N-acétyl-β-D-2-désoxy-2-amino-glucopyrannosyle ; ou un atome d'hydrogène ;
R₂ est un groupe α-D-mannopyrannosyle;
sous réserve que R₁ ne représente un atome d'hydrogène que lorsque R représente un atome d'hydrogène ;
Y est COOH ;
et ses sels d'addition pharmaceutiquement acceptables ; qui comprend le fait de soumettre un dérivé de teicoplanine de formule I, dans laquelle R et R₁ sont tels que définis ci-dessus et R₂ est un atome d'hydrogène, à une transformation microbiologique avec une culture d'Actinoplanes teichomyceticus ATCC 31121, ses mutants naturels ou ses variants présentant la même propriété de former la liaison glycosidique entre le fragment de D-mannose et la fonction hydroxylique de la position -OR₂ de la teicoplanine constituant la matière de départ, le mycélium lavé ou une préparation acellulaire correspondants et la récupération de ladite substance ou d'un mélange de substances et, dans le cas où on obtient un mélange de substances, éventuellement sa séparation en ses composants individuels.

2. Procédé selon la revendication 1, où la transformation microbienne est réalisée par contact du substrat avec une culture en croissance de la souche ci-dessus, cultivée dans des conditions aérobies submergées, dans un milieu contenant des sources assimilables de carbone, d'azote et de sels minéraux, à une température comprise entre 25°C et 35°C pendant une période de 4 à 48 heures, et la récupération des substances et/ou la séparation éventuelle de leur mélange sont effectuées selon des modes de séparation et de purification connus en soi dans la technique.

3. Procédé selon les revendications 1 et 2, où on utilise un mycélium de la culture du microorganisme de mannosylation identifié ci-dessus, lavé dans une solution salée isotonique.

4. Composé de formule I dans laquelle R et R₁ sont des atomes d'hydrogène, R₂ est un groupe α-D-mannopyrannosyle et Y est COOH.

5. Composé selon la revendication 4 pour l'utilisation comme médicament.

6. Utilisation du composé selon la revendication 4 comme facteur de croissance des animaux.

7. Composition pharmaceutique comprenant une quantité efficace du point de vue antibactérien, du composé antibiotique de la revendication 4.

8. Composition alimentaire pour animaux comprenant une quantité non toxique de l'antibiotique de la revendication 4 qui est efficace comme promoteur de la croissance.
